# EUROPEAN PATENT APPLICATION

(11) **EP 1 994 928 A1**
(43) Date of publication of application: **26.11.2008**
(21) Application number: 07737998.0
(22) Date of filing: 08.03.2007
(51) Int. Cl.: A61K 31/11, A01N 35/04, A01N 43/16, A01N 43/40, A61K 31/353, A61K 31/4406, A61P 31/04, A61P 31/10, A23G 3/00, A23G 3/34, A61K 8/34, A61K 8/49, A61K 8/97, A61K 36/00, A61Q 11/00

(54) **ANTIMICROBIAL AGENT AND ANTIMICROBIAL COMPOSITION**

(30) Priority: 10.03.2006 JP 2006066394
(71) Applicant: Tsuchida, Yuuzou, Tokyo 1420062 (JP)
(72) Inventor: TSUCHIDA, Kotarou, Shinagawa-ku, Tokyo 1420062 (JP); WATANABE, Kunitomo, Gifu-shi, Gifu 5020816 (JP); SAKURAI, Daisuke, Shinagawa-ku, Tokyo 1420062 (JP); KOKETSU, Mamoru, Gifu-shi, Gifu 5020813 (JP); KAWABE, Mitsuo, Kawaguchi-shi, Saitama 3340056 (JP); UTSUMI, Teruo, Shibuya-ku, Tokyo 1510064 (JP)
(74) Representative: Müller-Boré & Partner Patentanwälte
(86) International application number: PCT/JP2007/054507
(87) International publication number: WO 2007/105581

(57) **Abstract**

A microbicidal agent comprising as an effective ingredient at least one selected from the group consisting of p-hydroxybenzaldehyde, 5,7,4'-trihydroxy-3',5'-dimethoxyflavone, 3-hydroxypyridine and vanillin, and a microbicidal composition comprising the microbicidal agent.

## Description

### Technical Field

The present invention relates to a microbicidal agent and a microbicidal composition.

### Background Art

It is known for a long time that a bamboo (Sasa) extract has microbicidal activities. For example, it has been reported that the extract has a microbicidal effect against *Staphylococcus aureus, Pseudomonas aeruginosa* and *Escherichia coli,* which are the causative bacteria of wound infection (Patent Documents 1 and 2), as well as against *Helicobacter pylori* which is thought to be the causative bacteria of gastric ulcer.
Kumazasa is a plant belonging to family Gramineae, genus Sasa. In general, Sasa growing in mountains are called Kumazasa, which is a generic term of Sasas whose leaf margin becomes blighted and whitened in winter. There are various Sasas called Kumazasa, some of which have the scientific name "*Sasa albo marginata* Makino et Shibata" and the others have different names therefrom. Kumazasa (*Sasa albo marginata*) is now grown in almost all over Japan, because people spread it.

It is known for a long time that Kumazasa (*Sasa albo marginata*) exhibits an extremely wide variety of actions comparable to *Gymnema sylvestre* from India, guava from China and *Lycium chinense* from South Korea, which are known as a Chinese herbal remedy used in the Oriental medicine. Also in Japan, Kumazasa has been used as a traditional ethnomedical for gastropathy, diabetes mellitus, hypertension and so on (Non-patent Documents 1-3), as well as used as a food packaging material for e.g. Sasa-dango or Chimaki, or as an analeptic.
The animal experiments done in postwar days revealed that Kumazasa had an inhibitory activity against hepatoma in mice, and some pharmacological studies have been carried out from the viewpoint of carcinostatic effect (Non-patent Document 4). Further, studies about an excellent preservative activity (Non-patent Document 5) and microbicidal activity (Non-patent Document 6) which Kumazasa has have also been carried out. However, in most of these studies, nothing more than the analysis of organic acids by using gas chromatography has been reported. There are few reports which disclose the isolation and purification of the essential components which provide a microbicidal effect.
It has also been known that coumaric acid and derivatives thereof have a microbicidal activity against *Escherichia coil, Staphylococcus aureus* and *Pseudomonas aeruginosa* (Patent Document 3).
Moreover, it has also been known that Kumazasa extract contains phenylpropanoids such as coumaric acid, ferulic acid, coffeic acid and vanillin, 3-hydroxypyridine and the like, and a mixture of these shows a microbicidal activity against *Escherichia coil, Staphylococcus aureus* and *Pseudomonas aeruginosa* (Patent Document 4).

Patent Document 1: WO 00/067707
Patent Document 2: JP 2003-201247 A
Patent Document 3: JP 2004-359626 A
Patent Document 4: JP 2006-36731 A
Non-patent Document 1: M. Shibata, K. Kubo, M. Onoda, Journal of the Pharmaceutical Society of Japan, 98, 1436 (1978).
Non-patent Document 2: S. Okabe, K. Takeuchi, K. Takagi, M. Shibata, Jpn. J. Pharmacol., 25, 608 (1975).
Non-patent Document 3: M. Shibata, F. Sato, K. Takeshita, K. Otani, Natural Medicines (Shoyaku-gaku zasshi), 34, 274 (1980)
Non-patent Document 4: M. Shibata, K. Kubo, M. Onoda, Folia Pharmacol. Jpn, 72, 531-541 (1976)
Non-patent Document 5: N. V. Chuyen, T. Kurata, H. Kato, J. Antibact. Antifung. Agents, 11, 69-75 (1983)
Non-patent Document 6: N. V. Chuyen, H. Kato, Agric. Biol. Chem., 46, 2795-2801(1982)3-1128 (2004)

### Disclosure of the Invention

### Problems to be solved by the Invention

An object of the present invention is to provide a microbicidal agent.
Another object of the present invention is to provide a microbicidal composition comprising the microbicidal agent.

### Means for Solving Problem

The present invention provides a microbicidal agent and a microbicidal composition comprising the same as follows.
1. A microbicidal agent comprising as an effective ingredient at least one selected from the group consisting of p-hydroxybenzaldehyde, 5,7,4'-trihydroxy-3',5'-dimethoxyflavone, 3-hydroxypyridine, and vanillin.
2. The microbicidal agent according to item 1 described above, wherein said microorganism is selected from the group consisting of *Streptococcus, Enterococcus, Staphylococcus, Escherichia, Salmonella, Yersinia, Vibrio, Pseudomonas, Bacillus,* and *Candida.*
3. A microbicidal agent comprising as an effective ingredient at least one selected from the group consisting of p-hydroxybenzaldehyde, and 5,7,4'-trihydroxy-3',5'-dimethoxyflavone.
4. The microbicidal agent according to item 3 described above, wherein said microorganism is selected from the group consisting of *Streptococcus, Enterococcus, Staphylococcus, Escherichia, Salmonella, Yersinia, Vibrio, Pseudomonas, Bacillus,* and *Candida.*
5. The microbicidal agent according to item 1 described above, wherein said agent comprises 3-hydroxypyridine as an effective ingredient, and said microorganism is selected from the group consisting of *Streptococcus pneumoniae, Streptococcus pyogenes, Staphylococcus aureus, Yersinia enterocolitica, Pseudomonas aeruginosa,* and *Candida* sp..
6. The microbicidal agent according to item 1 described above, wherein said agent comprises p-hydroxybenzaldehyde as an effective ingredient, and said microorganism is selected from the group consisting of *Streptococcus pneumoniae, Streptococcus pyogenes, Staphylococcus aureus, Escherichia coli, Salmonella* sp., *Yersinia enterocolitica, Vibrio parahaemolyticus, Pseudomonas aeruginosa, Bacillus cereus, Bacillus subtilis,* and *Candida* sp..
7. The microbicidal agent according to item 1 described above, wherein said agent comprises vanillin as an effective ingredient, and said microorganism is selected from the group consisting of *Streptococcus pneumoniae, Streptococcus pyogenes, Staphylococcus aureus, Escherichia coli, Salmonella* sp., *Yersinia enterocolitica, Vibrio parahaemolyticus, Bacillus cereus, Bacillus subtilis,* and *Candida* sp..
8. The microbicidal agent according to item 1 described above, wherein said agent comprises 5,7,4'-trihydroxy-3',5'-dimethoxyflavone as an effective ingredient, and said microorganism is selected from the group consisting of *Salmonella* sp., and *Pseudomonas aeruginosa.*
9. A microbicidal composition comprising the microbicidal agent according to any one of items 1 to 8 described above.
The term "microorganism" used herein includes bacteria, mycobacteria, cyanobacteria, archaebacteria, fungi, yeasts, algae, viruses and the like, and particularly the term means microorganisms which have an undesirable effect on animals and plants including human.

### Effects of the Invention

The microbicidal agent according to the present invention has a high microbicidal activity against various microorganisms, particularly *Streptococcus, Enterococcus, Staphylococcus, Escherichia, Salmonella, Yersinia, Vibrio, Pseudomonas, Bacillus, Candida* and so on; more specifically, *Streptococcus pneumoniae, Streptococcus pyogenes, Enterococcus faecalis, Staphylococcus aureus, Escherichia coli, Salmonella* sp., *Yersinia enterocolitica, Vibrio parahaemolyticus, Pseudomonas aeruginosa, Bacillus cereus, Bacillus subtilis,* and *Candida* sp..

### Best Mode for Carrying Out the Invention

In order to clarify the nature of the microbicidal effect of Kumazasa (*Sasa albo marginata*) and to specify the components effective for suppressing bacteria, the present inventors fractionated a hot water extract of Kumazasa leaf by using various solvents. After fractionating it by silica gel chromatography, they performed separation by using TLC as well as by using a preparative ODS column for high performance liquid chromatography to achieve isolation and purification. Further, they determined the structure of the isolated compounds by using various spectrum, and carried out microbicidal tests by using 13 kinds of bacteria. As a result, they discovered that the above-described p-hydroxybenzaldehyde, 5,7,4'-trihydroxy-3',5'-dimethoxyflavone (hereinafter also referred to as "tricin"), 3-hydroxypyridine, *trans*-p-coumaric acid (*trans*-p-hydroxycinnamic acid), and vanillin exhibit specific microbicidal activities against various microorganisms, thereby completing the present invention.

The present invention will now be described concretely.
As an experimental material, the hot water extract of Kumazasa (*Sasa albo marginata* Makino et Shibata) leaf obtained from Hououdou Co., Ltd. (Trade Name: TWEBS) was used. The solvent in 10 g of the hot water extract (hereinafter referred to as "Sa") was removed by an evaporator to obtain 6.2 g of solid content, indicating that the hot water extract of Kumazasa leaf, the source material, contained 38% water.
This hot water extract was subjected to fractionation by using various solvents. That is, the hot water extract (Sa) was extracted with ethyl acetate, and the solvent in the obtained ethyl acetate layer was evaporated, thereby obtaining an ethyl acetate-soluble fraction (hereinafter referred to as "Sa-1"). The aqueous layer was further extracted with n-butanol, and the same treatment was performed, thereby obtaining an n-butanol-soluble fraction and an aqueous layer. The ethyl acetate-soluble fraction (Sa-1), n-butanol-soluble fraction and aqueous layer which were obtained by these treatments, and the source material hot water extract (Sa) were subjected to a microbicidal test against *Staphylococcus aureus.* The cup method was employed in this test. In the cup method, a cup having a diameter of 7.32 mm is placed on the center of a medium on which bacteria grow. The sample solution is then dropped into the cup, and the sample solution spreads concentrically from the cup. If the sample has a microbicidal activity, the inhibition ring which indicates the inhibition of bacterial growth is formed. There is a positive correlation between the microbicidal activity and the size of the inhibition ring, hence the degree of a microbicidal activity can be judged based on the size of the inhibition ring. The results of the microbicidal test against *Staphylococcus aureus* on each fraction are shown in Table 1.

**Table 1**

| Experimental Plot | Diameter of Inhibition Ring (mm) | Judgement |
|---|---|---|
| Blank | 7.32 | - |
| Source Material (Sa) | 12.81 | ++++ |
| Ethyl Acetate-Soluble Fraction (Sa-1) | 12.23 | ++++ |
| n-Butanol Soluble Fraction | 8.37 | + |
| Aqueous Layer | 9.64 | ++ |

| | | |
|---|---|---|
| "Judgement" represents the degree of the microbicidal activity as follows: ++++, very strong; ++, the activity shown; +, slightly shown; -, no activity. "7.32 mm" in Blank indicates the diameter of the cup. | | |

According to the microbicidal test, the ethyl acetate-soluble fraction had a microbicidal activity almost equivalent to the hot water extract of Kumazasa. On the other hand, the microbicidal activity of the n-butanol-soluble fraction and the aqueous layer fraction was slight.
The above-described microbicidal test revealed that the microbicidal activity was distributed in the ethyl acetate-soluble fraction. Therefore, 2.3 kg of the hot water extract of Kumazasa leaf (Sa) was extracted with ethyl acetate. The solvent in the obtained ethyl acetate layer was evaporated to obtain 78 g of ethyl acetate-soluble fraction (Sa-1).
The microbicidal activity of the obtained Sa-1 against *Staphylococcus aureus* was assessed. As a result, it showed a microbicidal activity.

### Microbicidal Activity against Staphylococcus aureus

### Fractionation and Purification of Components Having Microbicidal Activity

### Fractionation of Ethyl Acetate-Soluble Fraction (Sa-1)

By using neutral silica gel chromatography, 52 g of the ethyl acetate-soluble fraction (Sa-1) which showed a microbicidal activity was fractionated. For the column elution, the step wise method in which the mixing ratio of the solvents were changed in steps was employed. The used solvents were, n-hexane/ethyl acetate = 5:5 in an amount of 2.5 L, and 4:6, 3:7, 2:8 each in an amount of 1 L, and methanol alone in an amount of 2.5 L. The separation of the eluted components was performed based on the result of TLC, and the detection of spots thereon was performed by using UV 254 nm. As a result, the ethyl acetate-soluble fraction was fractionated into 7 fractions. These 7 fractions were named, respectively, Sa-1-A (90 mg), Sa-1-B (250 mg), Sa-1-C (3.36 g), Sa-1-D (3.00 g), Sa-1-E (110 mg), Sa-1-F (1.56 g), Sa-1-G (30.0 g).

First, Sa-1-C (3.36 g) was dissolved in 5 mL of chloroform, and n-hexane in an amount equal to chloroform was added thereto, followed by leaving the mixture to stand overnight. As a result, the mixture was separated into two layers. The upper layer (hereinafter referred to as "Sa-1-C(1)") and the lower layer (hereinafter referred to as "Sa-1-C(2)") were recovered separately. The amount of Sa-1-C(1) was 2.22 g after removing the solvent, and the amount of Sa-1-C(2) was 1.14 g.

### Analysis of Sa-1-C(1)

To 2.22 g of Sa-1-C(1), 3.50 g of silica gel was added and the components therein were adsorbed, followed by carrying out the fractionation by using silica gel column chromatography. For the column elution, the step wise method was employed. The composition of the solvents used was, chloroform/acetone = 20:1 in an amount of 2 L, and 7:3, 6:4 each in an amount of 100 mL, and acetone alone in an amount of 500 mL. For recovery, 110 vials of 50 mL volume were used. Among 110 vials of 50 mL volume, vials No. 21 and 22 which had a spot in the same position according to TLC were mixed, and the resulting mixture was concentrated to obtain a concentrate (hereinafter referred to as "Sa-1-C(1)-a"). The concentrate was dissolved in methanol (0.5 mL) and the fractionation was tried by using a preparative ODS column for high performance liquid chromatography (HPLC).

### HPLC Separation of the Concentrate (Sa-1-C(1)-a) from Vials No. 21 and 22

Elution condition: Column: Wakosil-II 5C18HG prep, ϕ20.0 mm×250 mm, Mobile phase: 20% aqueous acetonitrile solution, Flow rate: 5 mL/min, Column temperature: 30°C, Detection: UV 260 nm
The major peak at 34.5 min was separated to obtain 60 mg of white solid (hereinafter referred to as "Sa-1-C(1)-aa"). By using deuterated methanol, ¹H and ¹³C NMR spectrum of this white solid (Sa-1-C(1)-aa) were measured.
¹H and ¹³C NMR Spectrum of White Solid (Sa-1-C(1)-aa)
¹H NMR (500 MHz, CD₃OD) : δ 3.91 (s, 3H), 6.94 (d, J = 8.6 Hz, 1H), 7.42 (d, J = 8.6 Hz, 1H), 7.43 (s, 1H), 9.74 (s, 1H)
¹³C NMR (125 MHz, CD₃OD): δ56.3, 111.2, 116.3, 127.9, 130.6, 149.6, 154.6, 192.8
IR (KBr) : 3179, 1668 cm⁻¹
mp : 114-115°C

A signal of a hydrogen at δ9.74 was observed in ¹H NMR spectrum and a signal at δ192.8 was observed in ¹³C NMR spectrum, indicating that there was an aldehyde group. A signal at δ3.91 was observed in ¹H NMR spectrum and a signal at δ56.3 was observed in ¹³C NMR spectrum, indicating that there was an methoxy group. Further, signals corresponding to three hydrogens at δ6.94 (d, J = 8.6 Hz, 1H) and δ7.42 (d, J = 8.6 Hz, 1H), δ7.43 (s, 1H), and six signals within δ111.2-154.6, originated from aromatic ring, were observed in ¹³C NMR spectrum. In IR spectrum, a characteristic absorption at 3179 cm⁻¹ for hydroxyl group was observed. These results indicated that the structure of the white solid (Sa-1-C(1)-aa) was deduced to be 3-hydroxy-4-methoxybenzaldehyde (vanillin). Because vanillin was a known compound, the data were compared to the literature value (The Aldrich Library of 13C and 1H FT NMR Spectra EDITION 1 Volume 2, Aldrich Chemical Company, Inc.). Further, because there was an possibility that the compound was an isomer isovanillin, the white solid (Sa-1-C(1)-aa) was compared to vanillin and isovanillin which were purchased as reagent by using an ODS column for high performance liquid chromatography (HPLC), thereby determining that the white solid (Sa-1-C(1)-aa) was vanillin.

Next, among 110 vials of 50 mL volume, vials No. 48-55 which had a spot in the same position according to TLC were mixed, and the resulting mixture was concentrated to obtain 152 mg of concentrate (hereinafter referred to as "Sa-1-C(1)-b"). This was fractionated by using silica gel column chromatography again. For the column elution, the step wise method was employed. The solvents used were, first chloroform alone in an amount of 200 mL, then chloroform/acetone = 9.5:0.5 in an amount of 200 mL, 9:1, 8:2, 7:3 and acetone alone each in an amount of 100 mL, and finally methanol alone in an amount of 200 mL. For recovery, 42 vials of 50 mL volume were used.
Among 42 vials of 50 mL volume, vials No. 23-33 which had a spot in the same position according to TLC were mixed, and the resulting mixture was concentrated to obtain 102 mg of concentrate (hereinafter referred to as "Sa-1-C(1)-ba"). The concentrate was purified by using preparative TLC. Chloroform/acetone = 7/3 was used as a developing solvent, and UV 254 nm was used for detection. The recovered silica gel was subjected to elution with acetone, and the eluent was subjected to suction filtration, followed by evaporation of the solvent to obtain 86 mg of light yellow solid (hereinafter referred to as "Sa-1-C(1)-baa"). By using deuterated methanol, ¹H and ¹³C NMR spectrum of this light yellow solid (Sa-1-C(1)-aa) were measured.

¹H and ¹³C NMR Spectrum of Light Yellow Solid (Sa-1-C(1)-baa)
¹H NMR (500 MHz, CD₃OD): δ6.92 (d, J = 8.4 Hz, 2H), 7.77 (d, J = 8.4 Hz, 2H), 9.76 (s, 1H)
¹³C NMR (125 MHz, CD₃OD): δ116.8, 130.2, 133.4, 165.1, 192.8
IR (KBr) : 3168, 1670 cm⁻¹
mp : 118-119°C

A signal of hydrogen at δ9.76 was observed in ¹H NMR spectrum and a signal at δ192.8 was observed in ¹³C NMR spectrum, indicating that there was an aldehyde group. The 8.6 Hz doublet signal corresponding to two hydrogens at δ6.92, 7.77 was observed in ¹H NMR spectrum, and four signals within δ116.8-165.1, originated from aromatic, was observed in ¹³C NMR spectrum, indicating that there was an aromatic ring having substitution at para-position. Further, in IR spectrum, a characteristic absorption at 3168 cm⁻¹ for hydroxyl group was observed. From these results, the light yellow solid (Sa-1-C(1)-baa) was thought to be p-hydroxybenzaldehyde having an aldehyde group and hydroxyl group at its para-position. The data were compared to the literature value (The Aldrich Library of 13C and 1H FT NMR Spectra EDITION 1 Volume 2, Aldrich Chemical Company, Inc.) to determine that the light yellow solid (Sa-1-C(1)-baa) was p-hydroxybenzaldehyde.

Analysis of Sa-1-C(2)
Recrystallization (acetone/hexane) of 1.14 g of Sa-1-C(2) was carried out twice to obtain 478 mg of light yellow-green solid (hereinafter referred to as "Sa-1-C(2)-a"). By using deuterated methanol, ¹H and ¹³C NMR spectrum of this light yellow-green solid (Sa-1-C(2)-a) were measured.
¹H and ¹³C NMR Spectrum of Light Yellow-Green Solid (Sa-1-C(2)-a)
¹H NMR (500 MHz, CD₃OD): δ6.28 (d, J = 16.0 Hz, 1H), 6.81 (d, J = 8.6 Hz, 2H), 7.45 (d, J = 8.6 Hz, 2H), 7.61 (d, J = 16.0 Hz, 1H)
¹³C NMR (125 MHz, CD₃ OD) : δ115.6, 116.8, 127.2, 131.1, 146.7, 161.1, 171.0
IR (KBr) : 3168, 2830, 975 cm⁻¹
mp : 213-214°C

Two doublet signals corresponding to four hydrogens at δ6.81 (d, J = 8.6 Hz, 2H) and 7.45 (d, J = 8.6 Hz, 2H) were observed in ¹H NMR spectrum, and two strong signals corresponding to two carbons at δ115.6 and 131.1 were observed in ¹³C NMR spectrum, indicating that this compound had an aromatic ring having substitution at para-position. Further, in ¹H NMR spectrum, two hydrogens having coupling constant of 16.0 Hz were observed at δ6.28 (d, J = 16.0 Hz, 1H) and 7.61 (d, J = 16.0 Hz, 1H), respectively, indicating that there was a pair of alkenes located in trans-position each other. The signal at δ171.0 in ¹³C NMR revealed the existence of a carbonyl group. In IR spectrum, a characteristic absorption at 3168 cm⁻¹ for hydroxyl group was observed. From these results, the structure of the light yellow-green solid (Sa-1-C(2)-a) was thought to be *trans*-p-coumaric acid. The data were compared to the literature value (The Aldrich Library of 13C and 1H FT NMR Spectra EDITION 1 Volume2, Aldrich Chemical Company, Inc.) to determine that the light yellow-green solid (Sa-1-C(2)-a) was *trans*-p-coumaric acid.

### Analysis of Sa-1-F

To Sa-1-F (1.56 g), which was one of the fractions obtained by fractionating the ethyl acetate-soluble fraction by silica gel column chromatography, chloroform was added and the resulting mixture was thoroughly stirred, followed by removing the liquid layer comprising components which dissolved in chloroform. Thereafter, acetone was added to the residue which did not dissolve in chloroform, and the resulting mixture was separated into an acetone-soluble liquid layer (hereinafter referred to as "Sa-1-F(1)") and an acetone-insoluble solid layer (hereinafter referred to as "Sa-1-F(2)"). Sa-1-F(1) was in an amount of 162 mg after evaporating the solvent, and Sa-1-F(2) was in an amount of 1.40 g.

### Analysis of Sa-1-F(1)

Acetone was added to 162 mg of Sa-1-F(1) to dissolve it again, and the resulting mixture was left to stand. Thereafter, only a clear upper portion of the liquid layer was recovered. The acetone solvent was evaporated to obtain 10 mg of yellow solid (hereinafter referred to as "Sa-1-F(1)-a"). The purity of this compound was confirmed by using an ODS column for high performance liquid chromatography (HPLC) to find that the purity was very high. It was found that the compound showed a strong absorption at UV 260 nm.
By using deuterated acetone, ¹H and ¹³C NMR spectrum of this yellow solid (Sa-1-F(1)-a) were measured.

¹H and ¹³C NMR Spectrum of Yellow Solid (Sa-1-F(1)-a)
¹H NMR (500 MHz, (CD₃ )₂ CO) : δ3.97 (s, 6H), 6.26 (d, J = 2.3 Hz, 1H), 6.56 (d, J = 2.3 Hz, 1H), 6.74 (s, 1H), 7.39 (s, 2H)
¹³C NMR (125 MHz, (CD₃)₂CO): δ56.9, 94.9, 99.7, 104.7, 105.2, 105.4, 122.4, 140.9, 149.1, 158.8, 163.4, 164.9, 165.1, 183.1
IR (KBr) : 3357, 1615 cm⁻¹
mp : 276-277°C
MS (FAB) : m/z = 331 [M⁺+1]

The ¹³C NMR spectrum revealed that there were 17 carbons, and the DEPT revealed that there were 2 primary carbons, 5 tertiary carbons, and 10 quaternary carbons. A singlet signal corresponding to 6 hydrogens at δ3.97 (s, 6H) was observed in ¹H NMR spectrum, and a signal at δ56.9 was observed in ¹³C NMR, indicating that there were two equivalent methoxy groups. In IR spectrum, the existence of a hydroxyl group at 3357 cm⁻¹ and an absorption for a carbonyl group at 1615 cm⁻¹ which possibly had a hydrogen bond were observed. Mass spectrometry revealed that the molecular weight was 330, suggesting that the compound was C₁₇H₁₄O₇. The yellow solid (Sa-1-F(1)-a) was thought to be 5,7,4'-trihydroxy-3',5'-dimethoxyflavone by the below-described formula. The data were compared to the literature value (C. Kong, X. Xu, B. Zhou, F. Hu, C. Zhang, M. Zhang, Phytochemistry, 65, 1123-1128 (2004)) to find that they were close to the literature value, thereby determining that this yellow solid (Sa-1-F(1)-a) was 5,7,4'-trihydroxy-3',5'-dimethoxyflavone.

### Analysis of Sa-1-F(2)

To 1.40 g of Sa-1-F(2), 3.00 g of silica gel was added and the components therein were adsorbed, followed by carrying out the fractionation by using silica gel column chromatography. For the column elution, the step wise method was employed. The used solvents were, chloroform/acetone = 10:1, 8:2 in an amount of 200 mL, 5:5, 3:7 and acetone alone each in an amount of 300 mL, and methanol alone in an amount of 200 mL. For recovery, 53 vials of 50 mL volume were used.
Among 53 vials of 50 mL volume, vials No. 42-48 which had a spot in the same position according to TLC were mixed, and the resulting mixture was concentrated to obtain 46 mg of light yellow solid (hereinafter referred to as "Sa-1-F(2)-a"). By using deuterated methanol, ¹H and ¹³C NMR spectrum of this light yellow solid (Sa-1-F(2)-a) were measured.

¹H and ¹³C NMR Spectrum of Light Yellow Solid (Sa-1-F(2)-a)
¹H NMR (500 MHz, CD₃OD) : δ 7.21-7.28 (m, 2H), 7.99 (dd, J = 4.1.0.7 Hz, 1H), 8.09 (d, J = 2.3 Hz, 1H)
¹³C NMR (125 MHz, CD₃OD) : δ 124.4, 125.9, 138.3, 140.8, 155.9
IR (KBr) : 3449 cm⁻¹
mp : 127-128°C

Signals corresponding to four hydrogens at δ7.21-7.28 (m, 2H), δ7.99 (dd, J = 4.1.0.7 Hz, 1H), δ8.09 (d, J = 2.3 Hz, 1H) were observed in ¹H NMR spectrum, and five signals within δ124.4-155.9, originated from aromatic ring, were observed in ¹³C NMR spectrum. A characteristic absorption at 3168 cm⁻¹ for hydroxyl group was observed in IR spectrum. From these results, the light yellow solid (Sa-1-F(2)-a) was thought to be 3-hydroxypyridine. The data were compared to the literature value (The Aldrich Library of 13C and 1H FT NMR Spectra EDITION 1 Volume2, Aldrich Chemical Company, Inc.). Considering the fact that the data were close to the literature value and its melting point (literature value: 126-129°C), the light yellow solid (Sa-1-F(2)-a) was determined to be 3-hydroxypyridine.

### Microbicidal Test

### Agar Dilution Method

As microbicidal test method for *trans*-p-coumaric acid, 3-hydroxypyridine, p-hydroxybenzaldehyde and vanillin, the agar dilution method was employed.
In agar dilution method, a solution in which a sample is dissolved is serially diluted several times, and each of the diluted sample solutions is mixed with agar medium to prepare plate agars having various sample concentration. Then bacteria is inoculated onto the plate agars. In cases where the sample has a microbicidal activity, the growth of the bacteria on the plates can be inhibited. The lowest concentration which can inhibit the growth of the bacteria is referred to as Minimum Inhibitory Concentration (MIC), thereby judging the degree of a microbicidal activity of the sample.
First, 400 mg of sample was dissolved in 1 mL of DMSO to prepare 400 mg/mL sample solution. Then 0.5 mL of this 400 mg/mL sample solution was diluted 2-fold to prepare 200 mg/mL sample solution. In the same manner, sample solutions of 100 mg/mL, 50.0 mg/mL, and 25.0 mg/mL were prepared. In addition, 300 mg/mL sample solution was prepared. Thereafter, 0.2 mL of these sample solutions each was mixed with 19.8 mL of BHI (Brain Heart Infusion Agar, Becton Dickinson, MD) medium, thereby preparing agar plates having the sample concentration of 4.0 mg/mL, 3.0 mg/mL, 2.0 mg/mL, 1.0 mg/mL, 0.5 mg/mL, and 0.25 mg/mL in dishes. As a control for comparison of microbicidal activity of samples, agar plates containing catechin having the above-described concentrations were prepared. Sterilized water was used for dissolving catechin, because catechin was insoluble in DMSO.

As test bacteria, 13 kinds of bacteria, i.e., *Streptococcus pneumoniae, Streptococcus pyogenes, Enterococcus faecalis, Staphylococcus aureus, Escherichia coli, Salmonella* sp., *Yersinia enterocolitica, Vibrio parahaemolyticus, Pseudomonas aeruginosa, Bacillus cereus, Bacillus subtilis* MB-32, *Bacillus subtilis* ATCC6633, and *Candida* sp., were used. These bacteria each was mixed with 2 mL of sterilized MHB^{R} liquid medium (Mueller Hinton Broth^{R}, Becton Dickinson, MD) to prepare bacterial suspension having turbidity 1 (bacterial concentration 10⁸/mL). Previously, small test tubes were set into each cell of a grating and sterilized, and 1 mL of 13 bacterial suspensions were added into the small test tubes set in the grating, respectively: By using a microplanter (SAKUMA, model: MIT-P), 10 µL aliquot was collected from each test tube set in the grating and transferred to other test tube, and the transferred bacterial suspensions were diluted 100-fold (bacterial concentration 10⁶/mL).
By using the microplanter, 10 µL (bacterial number 14.0) of this bacterial suspension was inoculated onto the plates containing the sample solution of various concentrations. The plates were incubated for 18 hours in an incubator at 37°C, and then a microbicidal activity of each compound was assessed. The results are shown in Tables 2 to 6.

**Table 2**

| Results of Microbicidal Test of *trans*-p-Coumaric Acid +: grew, -: did not grow | | | | | | | | | |
|---|---|---|---|---|---|---|---|---|---|
| No | | Concentration (mg/mL) | | | | | | | MIC |
| | | 0 | 0.25 | 0.5 | 1.0 | 2.0 | 3.0 | 4.0 | |
| 1 | *Streptococcus pneumoniae* | + | + | + | + | - | - | - | 2.0 |
| 2 | *Streptococcus pyogenes* | + | + | + | + | + | - | - | 3.0 |
| 3 | *Enterococcus faecalis* | + | + | + | + | + | + | + | |
| 4 | *Staphylococcus aureus* | + | + | + | + | + | + | - | 4.0 |
| 5 | *Escherichia coli* | + | + | + | + | + | + | + | |
| 6 | *Salmonella* sp. | + | + | + | + | + | + | + | |
| 7 | *Yersinia enterocolitica* | + | + | + | + | + | + | - | 4.0 |
| 8 | *Vibrio trhacaemalyticuss* | + | + | + | + | + | - | - | 3.0 |
| 9 | *Pseudomonas aeruginosa* | + | + | + | + | + | + | + | |
| 10 | *Bacillus cereus* | + | + | + | + | + | - | - | 3.0 |
| 11 | *Bacillus subtilis* MB-32 | + | + | + | + | + | + | + | |
| 12 | *Bacillus subtilis* ATCC 6633 | + | + | + | + | + | - | - | 3.0 |
| 13 | *Candida* sp. | + | + | + | + | + | - | - | 3.0 |

**Table 3**

| Results of Microbicidal Test of 3-Hydroxypyridine +: grew, -: did not grow | | | | | | | | | |
|---|---|---|---|---|---|---|---|---|---|
| No | | Concentration (mg/mL) | | | | | | | MIC |
| | | 0 | 0.25 | 0.5 | 1.0 | 2.0 | 3.0 | 4.0 | |
| 1 | *Streptococcus pneumoniae* | + | + | + | + | + | + | - | 4.0 |
| 2 | *Streptococcus pyogenes* | + | + | + | + | + | + | - | 4.0 |
| 3 | *Enterococcus faecalis* | + | + | + | + | + | + | + | |
| 4 | *Staphylococcus aureus* | + | + | + | + | + | + | - | 4.0 |
| 5 | *Escherichia coli* | + | + | + | + | + | + | + | |
| 6 | *Salmonella* sp. | + | + | + | + | + | + | + | |
| 7 | *Yersinia enterocolitica* | + | + | + | + | + | + | - | 4.0 |
| 8 | *Vibrio trhacaemalyticuss* | + | + | + | + | + | + | - | 4.0 |
| 9 | *Pseudomonas aeruginosa* | + | + | + | + | + | + | - | 4.0 |
| 10 | *Bacillus cereus* | + | + | + | + | + | + | + | |
| 11 | *Bacillus subtilis* MB-32 | + | + | + | + | + | + | + | |
| 12 | *Bacillus subtilis* ATCC 6633 | + | + | + | + | + | + | + | |
| 13 | *Candida* sp. | + | + | + | + | + | + | - | 4.0 |

**Table 4**

| Results of Microbicidal Test of p-Hydroxybenzaldehyde +: grew, -: did not grow | | | | | | | | | |
|---|---|---|---|---|---|---|---|---|---|
| No | | Concentration (mg/mL) | | | | | | | MIC |
| | | 0 | 0.25 | 0.5 | 1.0 | 2.0 | 3.0 | 4.0 | |
| 1 | *Streptococcus pneumoniae* | + | + | + | + | - | - | - | 2.0 |
| 2 | *Streptococcus pyogenes* | + | + | + | + | - | - | - | 2.0 |
| 3 | *Enterococcus faecalis* | + | + | + | + | + | + | + | |
| 4 | *Staphylococcus aureus* | + | + | + | + | + | - | - | 3.0 |
| 5 | *Escherichia coli* | + | + | + | + | - | - | - | 2.0 |
| 6 | *Salmonella* sp. | + | + | + | + | - | - | - | 2.0 |
| 7 | *Yersinia enterocolitica* | + | + | - | - | - | - | - | 0.5 |
| 8 | *Vibrio trhacaemalyticuss* | + | + | + | + | - | - | - | 2.0 |
| 9 | *Pseudomonas aeruginosa* | + | + | + | + | - | - | - | 2.0 |
| 10 | *Bacillus cereus* | + | + | + | + | + | - | - | 3.0 |
| 11 | *Bacillus subtilis* MB-32 | + | + | + | + | + | + | - | 3.0 |
| 12 | *Bacillus subtilis* ATCC 6633 | + | + | + | + | + | - | - | 3.0 |
| 13 | *Candida* sp. | + | + | + | + | + | - | - | 3.0 |

**Table 5**

| Results of Microbicidal Test of Vanillin +: grew, -: did not grow | | | | | | | | | |
|---|---|---|---|---|---|---|---|---|---|
| No | | Concentration (mg/mL) | | | | | | | MIC |
| | | 0 | 0.25 | 0.5 | 1.0 | 2.0 | 3.0 | 4.0 | |
| 1 | *Streptococcus pneumoniae* | + | + | + | + | + | - | - | 3.0 |
| 2 | *Streptococcus pyogenes* | + | + | + | + | - | - | - | 2.0 |
| 3 | *Enterococcus faecalis* | + | + | + | + | + | + | + | |
| 4 | *Staphylococcus aureus* | + | + | + | + | + | + | - | 4.0 |
| 5 | *Escherichia coli* | + | + | + | + | + | - | - | 3.0 |
| 6 | *Salmonella* sp. | + | + | + | + | + | - | - | 3.0 |
| 7 | *Yersinia enterocolitica* | + | + | + | - | - | - | - | 1.0 |
| 8 | *Vibrio trhacaemalyticuss* | + | + | + | + | - | - | - | 2.0 |
| 9 | *Pseudomonas aeruginosa* | + | + | + | + | + | + | + | |
| 10 | *Bacillus cereus* | + | + | + | + | + | + | - | 4.0 |
| 11 | *Bacillus subtilis* MB-32 | + | + | + | + | + | + | - | 4.0 |
| 12 | *Bacillus subtilis ATCC 6633* | + | + | + | + | + | + | - | 4.0 |
| 13 | *Candida* sp. | + | + | + | + | + | - | - | 3.0 |

**Table 6**

| Results of Microbicidal Test of Catechin +: grew, -: did not grow | | | | | | | | | |
|---|---|---|---|---|---|---|---|---|---|
| No | | Concentration (mg/mL) | | | | | | | MIC |
| | | 0 | 0.25 | 0.5 | 1.0 | 2.0 | 3.0 | 4.0 | |
| 1 | *Streptococcus pneumoniae* | + | + | + | - | - | - | - | 1.0 |
| 2 | *Streptococcus pyogenes* | + | + | + | + | - | - | - | 2.0 |
| 3 | *Enterococcus faecalis* | + | + | + | + | + | + | + | |
| 4 | *Staphylococcus aureus* | + | + | + | - | - | - | - | 1.0 |
| 5 | *Escherichia coli* | + | + | + | + | + | + | + | 2.0 |
| 6 | *Salmonella* sp. | + | + | + | + | - | - | - | 2.0 |
| 7 | *Yersinia enterocolitica* | + | + | + | - | - | - | - | 1.0 |
| 8 | *Vibrio trhacaemalyticuss* | + | + | - | - | - | - | - | 0.5 |
| 9 | *Pseudomonas aeruginosa* | + | + | + | - | - | - | - | 1.0 |
| 10 | *Bacillus cereus* | + | + | + | - | - | - | - | 1.0 |
| 11 | *Bacillus subtilis* MB-32 | + | + | + | + | - | - | - | 2.0 |
| 12 | *Bacillus subtilis* ATCC 6633 | + | + | + | + | + | - | - | 3.0 |
| 13 | *Candida* sp. | + | + | + | - | - | - | - | 1.0 |

The above-described results showed that *trans*-p-coumaric acid had an activity against *Streptococcus pneumoniae, Streptococcus pyogenes, Staphylococcus aureus, Yersinia enterocolitica, Vibrio parahaemolyticus, Bacillus cereus, Bacillus subtilis* ATCC6633, *Candida* sp.. Its MICs were, 2.0 mg/mL against *Streptococcus pneumoniae,* 3.0 mg/mL against *Streptococcus pyogenes, Vibrio parahaemolyticus, Bacillus cereus, Bacillus subtilis* ATCC6633 and *Candida* sp., and 4.0 mg/mL against *Staphylococcus aureus* and *Yersinia enterocolitica.*
It was shown that 3-hydroxypyridine had an activity against *Streptococcus pneumoniae, Streptococcus pyogenes, Staphylococcus aureus, Yersinia enterocolitica, Pseudomonas aeruginosa,* and *Candida* sp.. Its MICs were 4.0 mg/mL against *Streptococcus pneumoniae, Streptococcus pyogenes, Staphylococcus aureus, Yersinia enterocolitica, Pseudomonas aeruginosa,* and *Candida* sp..

It was shown that p-hydroxybenzaldehyde had an activity against many kinds of bacteria, that is, *Streptococcus pneumoniae, Streptococcus pyogenes, Staphylococcus aureus, Escherichia coli, Salmonella* sp., *Yersinia enterocolitica, Vibrio parahaemolyticus, Pseudomonas aeruginosa, Bacillus cereus, Bacillus subtilis* MB-32, *Bacillus subtilis* ATCC6633, and *Candida* sp.. Particularly, p-hydroxybenzaldehyde showed a strong activity against *Yersinia enterocolitica,* and its MIC against *Yersinia* was 0.5 mg/mL.
It was shown that vanillin had an activity against *Streptococcus pneumoniae, Streptococcus pyogenes, Staphylococcus aureus, Escherichia coli, Salmonella* sp., *Yersinia enterocolitica, Vibrio parahaemolyticus, Bacillus cereus, Bacillus subtilis* MB-32, *Bacillus subtilis* ATCC6633, and *Candida* sp.. Vanillin also had an activity against many kinds of bacteria. In particular, among these, vanillin showed a strong activity against *Yersinia enterocolitica,* and its MIC against *Yersinia* was 0.5 mg/mL.

### Paper Disk Method

As microbicidal test method for 5,7,4'-trihydroxy-3',5'-dimethoxyflavone, the paper disk method was employed, by which method the microbicidal activity can be judged even in cases where a solvent has some influences.
In paper disk method, sample solutions having various sample concentration are prepared, and the sample solution was absorbed in a special paper disk, followed by drying the paper disk. Onto the plate which is made by mixing a prepared bacterial suspension with agar medium, the paper disks are placed. If the sample has a microbicidal activity, the inhibition ring which indicates the inhibition of bacterial growth is formed such that the center of the ring is coincident with the paper disk. The degree of a microbicidal activity is judged based on the size of the inhibition ring.
First, 4 mg of 5,7,4'-trihydroxy-3',5'-dimethoxyflavone sample was dissolved in 200 µL to prepare 20 mg/mL sample solution. To 100 µL of this 20 mg/mL sample solution, 100 µL of sterilized water was added to prepare 200 µL of 10 mg/mL sample solution. To the solution, 60 µL DMSO and 140 µL of sterilized water were added to prepare 400 µL of 5.0 mg/mL sample solution. DMSO was used in the concentration of 40%, because the sample precipitated from 30% DMSO. To 350 µL of 5.0 mg/mL sample solution, 140 µL DMSO and 210 µL of sterilized water were added to prepare 700 µL of 2.5 mg/mL sample solution. In the same manner, 1.25 mg/mL and 0.625 mg/mL sample solutions were prepared. As a control, a solution containing 40% DMSO and 60% sterilized water was prepared. As a control for comparison, aqueous catechin solutions having the above-described concentrations were prepared. In a paper disk (8.12 mm diameter), 70 µL of these solutions each was absorbed, and the paper disks were dried for a day.
As test bacteria, *Staphylococcus aureus, Salmonella* sp., *Yersinia enterocolitica, Pseudomonas aeruginosa,* and *Candida* sp. were used. These bacteria were mixed with 2 mL of sterilized water to prepare bacterial suspensions having turbidity 1 (10⁸/mL). The plate agar was made by mixing 0.2 mL of the bacterial suspension with 19.8 mL of BHI medium. The dried paper disk was placed onto the plate agar, and the plate was incubated in an incubator at 37°C, followed by assessing the activity. The results are shown in Table 7. In the Table, (-) represents that no microbicidal activity was observed.

**Table 7**

| | | 5,7,4'-Trihydroxy-3',5'-Dimethoxyflavone | | | | Catechin | | | |
|---|---|---|---|---|---|---|---|---|---|
| | | Concentration (mg/mL) | | | | Concentration (mg/mL) | | | |
| | | 0 | 0.625 | 1.25 | 2.5 | 0 | 0.625 | 1.25 | 2.5 |
| 1 | *Staphylococcus aureus* | - | - | - | - | - | - | - | - |
| 2 | *Salmonella sp.* | 12.3 | 12.3 | 13.82 | 16.65 | - | - | - | - |
| 3 | *Yersinia enterocolitica* | 15.65 | 15.67 | 15.76 | 15.76 | - | - | - | 9.49 |
| 4 | *Pseudomonas aeruginosa* | 12.33 | 15.46 | 18.54 | 19.20 | - | - | - | - |
| 5 | *Candida* sp. | 11.86 | 11.86 | 12.30 | 11.90 | - | - | - | - |

These results indicated that 5,7,4'-trihydroxy-3',5'-dimethoxyflavone had a microbicidal activity against *Salmonella* sp. and *Pseudomonas aeruginosa.* Its microbicidal activity was very strong even compared to catechin.

A hot water extract of Kumazasa leaf (Sa, 2.3 kg) was extracted with ethyl acetate to obtain an ethyl acetate-soluble fraction (Sa-1, 78 g). The microbicidal test against *Staphylococcus aureus* revealed that the microbicidal activity was distributed in the ethyl acetate-soluble fraction (Sa-1). The ethyl acetate-soluble fraction (Sa-1) was fractionated into 7 fractions by using neutral silica gel column chromatography. The 7 fractions were named Sa-1-A (90 mg), Sa-1-B (250 mg), Sa-1-C (3.36 g), Sa-1-D (3.00 g), Sa-1-E (110 mg), Sa-1-F (1.56 g), and Sa-1-G (30.0 g), respectively. Sa-1-C (3.36 g) was separated into two layers by adding thereto chloroform and n-hexane to obtain Sa-1-C(1) (2.22 g) and Sa-1-C(2) (1.14 g). Sa-1-C(1) (2.22 g) was fractionated by using neutral silica gel column chromatography to obtain Sa-1-C(1)-a and Sa-1-C(1)-b. Sa-1-C(1)-a was fractionated by using a preparative ODS column for high performance liquid chromatography (HPLC) to obtain 60 mg of white solid (Sa-1-C(1)-aa). The spectrum data and analysis by HPLC revealed that the white solid (Sa-1-C(1)-aa) was vanillin. Sa-1-C(1)-b was fractionated by using neutral silica gel column chromatography again to obtain Sa-1-C(1)-ba, and this Sa-1-C(1)-ba was subjected to preparative TLC to obtain 86 mg of light yellow solid (Sa-1-C(1)-baa). The spectrum data revealed that the light yellow solid (Sa-1-C(1)-baa) was p-hydroxybenzaldehyde. Further, Sa-1-C(2) (1.14 g) was subjected to recrystallization twice to obtain 478 mg of light yellow-green solid (Sa-1-C(2)-a). The spectrum data revealed that the light yellow-green solid (Sa-1-C(2)-a) was *trans*-p-coumaric acid. Next, Sa-1-F (1.56 g) was separated into acetone-soluble Sa-1-F(1) (162 mg) and acetone-insoluble Sa-1-F(2) (1.40 g). Acetone was again added to Sa-1-F(1) (162 mg), and the liquid layer was separated to obtain 10 mg of yellow solid (Sa-1-F(1)-a). The spectrum data revealed that the yellow solid (Sa-1-F(1)-a) was 5,7,4'-trihydroxy-3',5'-dimethoxyflavone. Further, Sa-1-F(2) (1.40 g) was subjected to neutral silica gel column chromatography to obtain 46 mg of light yellow solid (Sa-1-F(2)-a). The spectrum data revealed that the light yellow solid (Sa-1-F(2)-a) was 3-hydroxypyridine.

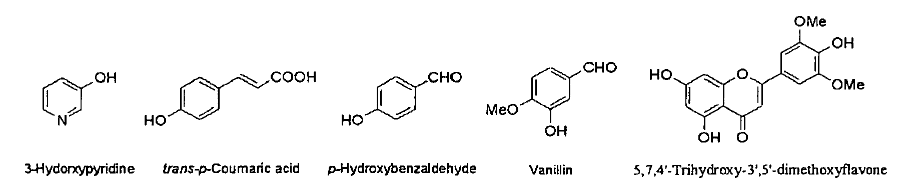

The microbicidal activity of vanillin, p-hydroxybenzaldehyde, *trans*-coumaric acid and 3-hydroxypyridine was examined by agar dilution method. As a result, it was confirmed that vanillin, p-hydroxybenzaldehyde, *trans*-coumaric acid and 3-hydroxypyridine had a microbicidal activity. Among these, vanillin and p-hydroxybenzaldehyde showed a strong microbicidal activity against *Yersinia enterocolitica,* the causative bacteria of food poisoning. For a microbicidal test of 5,7,4'-trihydroxy-3',5'-dimethoxyflavone, paper disk method was used. As a result, 5,7,4'-trihydroxy-3',5'-dimethoxyflavone showed a microbicidal activity against *Salmonella* sp. and *Pseudomonas aeruginosa.* Its activity was very strong even compared to catechin.

The present inventor tried to extract the above-described effective components from plants other than Kumazasa. As a result, 5,7,4'-trihydroxy-3',5'-dimethoxyflavone (tricin) existed abundantly in many plants belonging to Gramineae, for example, rice plant (rice), wheat, maize, barley, rye, sugarcane, bamboo (Take), Japanese pampas grass, pampas grass, reed (also called phragmites or Yoshi) and so on. Concrete plant names are as follows:
Rice plant, wheat, barley, *Avena fatua,* rye, proso millet, foxtail millet, Japanese barnyard millet, maize, finger millet, sorghum, Take, wild rice (Makomo), sugarcane, job's tears, reed, Japanese pampas grass, Sasa, *Arundo donax, Cortaderia argentea,* and lawn grass.

### Family Gramineae

### Subfamily Bambusioideae (Family Bambusaceae)

Genus *Bambusa:* Houraichiku (*Bambusa multiplex* (Lour.) Raeusch.), Hououchiku (*Bambusa multiplex* (Lour.) Raeusch. ex J.A. et J.H.Schult. 'Fernleaf') (hereinbefore mentioned belong to bamboo)
Genus *Shibataea:* Okamezasa (*Shibataea kumasasa* (Zoll. ex Steud.) Nakai
Genus *Phyllostachys*: Mousouchiku (*Phyllostachys heterocycla* (Carriere) Matsum.), Madake (*Phyllostachys bambusoides* Sieb. et Zucc.), Hachiku (*Phyllostachys nigra* (Lodd. ex Loud.) Munro var. *henonis* (Bean) Stapf ex Rendle)
Genus *Semiarundinaria*: Narihiradake (*Semiarundinaria fastuosa* (Mitford) Makino ex Nakai), Yashyadake (*Semiarundinaria yashadake* (Makino) Makino) (hereinbefore belong to Take)
Genus *Pleioblastus*: Nezasa (*Pleioblastus argenteostriatus* (Regel) Nakai f. *glaber* (Makino) Murata), Azumanezasa (*Pleioblastus chino* (Franch. et Sav.) Makino), Medake (*Pleioblastus Simonii* (Carr.) Nakai), Ryukyuchiku (*Pleioblastus linearis* (Hack.) Nakai)
Genus *Pseudosasa*: Yadake (*Pseudosasa japonica* (Siebold et Zucc. ex Steud.) Makino ex Nakai), Yakushimadake (*Pseudosasa owatarii* (Makino) Makino ex Nakai)
Genus *Sasamorpha*: Suzutake (*Sasamorpha borealis* (Hack.) Nakai)
Genus *Arundrinaria*: Azumazasa *(Arundinaria ramosa* Makino), Suekozasa (*Arundinaria ramosa* Makino var. *suwekoana* (Makino) Murata)
Genus *Sasa:* Miyakozasa (*Sasa nipponica* (Makino) Makino et Shibata), Chimakizasa (*Sasa palmata* (Lat.-Marl. ex Burb.) E.G.Camus), Kumazasa, Chishimazasa (*Sasa kurilensis* (Rupr.) Makino et Shibata) (hereinbefore mentioned belong to Sasa)

### Subfamily Poelideae

Genus *Agropyron*: Shibamugi (couch grass, *Agropyron repens* (L.) P.Beauv.), Kamojigusa (wheat grass, *Agropyron tsukushiense* (Honda) Ohwi var. *transiens* (Hack.) Ohwi), Aokamojigusa (*Agropyron ciliare* (Trin.) Franch. var. *minus* (Miq.) Ohwi)
Genus *Alopecurus*: Suzumeno-teppou (*Alopecurus aequalis* Sobol. var. *amurensis* (Kom.) Ohwi), Setogaya (*Alopecurus japonicus* Steud.)
Genus *Arundo:* Danchiku (*Arundo donax L.*)
Genus *Briza:* Kobansou (quaking grass, *Briza maxima* L.), Himekobansou (*Briza minor* L.)
Genus *Bromus:* Suzumeno-chahiki (bromegrass, *Bromus japonicus* Thunb.), Inumugi
(*Bromus catharticus* Vahl)
Genus *Dactylis*: Kamogaya (*Dactylis glomerata* L.)
Genus *Eragrostis*: Kazekusa (*Eragrostis ferruginea* (Thunb.) P.Beauv.), Suzumegaya (*Eragrostis cilianensis* (All.) Link ex Janchen), Shinadare-suzumegaya (*Eragrostis curvula* (Schrad.) Nees), Niwahokori (*Eragrostis multicaulis* Steud.)
Genus *Horudeum*: barley, Mugikusa (wall barley, *Hordeum murinum* L.)
Genus *Triticum:* wheat
Genus *Lolium*: Hosomugi (perennial rye grass, *Lolium perenne* L.), Nezumimugi (Italian rye grass, *Lolium multiflorum* Lam.), Dokumugi (bearded darnel, *Lolium temulentum* L.)
Genus *Poa*: Suzumeno-katabira (annual bluegrass, *Poa annua* L.), Ichigotsunagi (bluegrass, *Poa ochotensis* Trin.)
Genus *Festuca*: Naginatagaya (*Festuca myuros* L.), Ushinokegusa (fescue, *Festuca ovina* L.), Toboshigara (*Festuca parvigluma* Steud.)
Genus *Glyceria*: Mutsuoregusa (*Glyceria acutiflora* Torr. subsp. *japonica* T.Koyama et Kawano), Dojoutsunagi (*Glyceria ischyroneura* Steud.)
Genus *Melica*: Michishiba (*Melica onoei* Franch. et Sav.), Komegaya (*Melica nutans* L.)
Genus *Lophatherum:* Sasakusa (*Lophatherum gracile* Brongn.)
Genus *Leptochloa*: Azegaya (*Leptochloa chinensis* (L.) Nees)
Genus *Beckmannia*: Kazunokogusa (*Beckmannia syzigachne* (Steud.) Fernald)
Genus *Sporobolus*: Nezumino-o (*Sporobolus fertilis* (Steud.) Clayton), Higeshiba (*Sporobolus japonicus* (Steud.) Maxim. ex Rendle)
Genus *Eleusine*: Ohishiba (*Eleusine indica* (L.) Gaertn.), Shikokubie (finger millet, *Eleusine coracana* (L.) Gaertn.)
Genus *Cynodon*: Gyougishiba (Bermuda grass, *Cynodon dactylon* (L.) Pers.)
Genus *Phragmites*: Urahagusa (*Hakonechloa macra* (Munro ex S.Moore) Makino ex Honda), reed, Tsuruyoshi (*Phragmites japonica* Steud.)
Genus *Phaenosperma*
Genus *Leersia*
Genus *Oryza*: rice plant
Genus *Zizania*: Makomo (wild rice, *Zizania latifolia* (Griseb.) Turcz. ex Stapf)
Genus *Avena:* Karasumugi (*Avena fatua* L.), oat *(Avena sativa L.)*
Genus *Deshampsia*: Komesusuki (*Deschampsia flexuosa* (L.) Nees), Hirohano-komesusuki
(*Deschampsia cespitosa* (L.) P.Beauv. var. *festucifolia* Honda)
Genus *Trisetum:* Kanitsurigusa (*Trisetum bifidum* (Thunb.) Ohwi), Rishirikanitsuri (*Trisetum spicatum* (L.) K.Richt. subsp. *alascanum* (Nash) Hulten)
Genus *Koeleria*: Minoboro (*Koeleria cristata* (L.) Pers.)
Genus *Hierochloe*: Koubou (*Hierochloe glabra* Trin. subsp. *sachalinensis* (Printz) Tzvelev), Miyamakoubou (*Hierochloe alpina* (Sw.) Roem. et Schult)
Genus *Phalaris*: Kusayoshi (reed canary grass, *Phalaris arundinacea* L.), canary-kusayoshi (canary grass, *Phalaris canariensis* L.)
Genus *Agrostis*: Konukagusa (redtop, *Agrostis gigantea* Roth), Nukabo (*Agrostis clavata* Trin. subsp. *matsumurae* (Hack. ex Honda) Tateoka)
Genus *Polypogon*
Genus *Phleum*: Awagaeri (*Phleum paniculatum* Huds.), Ooawagaeri (timothy, *Phleum pratense* L.)
Genus *Calamagrostis*: Nogariyasu (*Calamagrostis brachytricha* Steud.), Hossugaya (*Calamagrostis pseudophragmites* (Hallier f.) Koeler), Yama-awa (*Calamagrostis epigeios* (L.) Roth)
Genus *Zoysia*: Shiba (lawn grass, *Zoysia japonica* Steud.), Kouraishiba (*Zoysia tenuifolia* auct. non Willd. ex Trin.), Onishiba (*Zoysia macrostachya* Franch. et Sav.), Nagamionishiba (*Zoysia sinica* Hance var. *nipponica* Ohwi)

### Subfamily Panicoideae

### Genus Arundinella

Genus *Eulalia*: Un-nuke (*Eulalia speciosa* (Debeaux) Kuntze), Un-nuke-modoki (*Eulalia quadrinervis* (Hack.) Kuntze)
Genus *Isachne*: Chigozasa (*Isachne globosa* (Thunb.) Kuntze), Haichigozasa (*Isachne nipponensis* Ohwi)
Genus *Panicum*: Kibi (proso millet, *Panicum miliaceum* L.), Haikibi (*Panicum repens* L.), Nukakibi (*Panicum bisulcatum* Thunb.)
Genus *Echinochloa*: Hie (Japanese barnyard millet, *Echinochloa esculenta* (A. Braun) H. Scholz), Inubie (*Echinochloa crus-galli* (L.) Beauv. var. *crus-galli*)
Genus *Oplismenus*: Chijimizasa (*Oplismenus undulatifolius* (Ard.) Roem. et Schult.)
Genus *Setaria*: Sasakibi (*Setaria palmifolia* (J.Koenig) Stapf), Enokorogusa (bristle grass, *Setaria viridis* (L.) P.Beauv.), Awa (foxtail millet, *Setaria italica* P.Beauv.), Kin-enokoro (*Setaria pumila* (Poir.) Roem. et Schult.)
Genus *Paspalum*: Suzumenohie (*Paspalum thunbergii* Kunth ex Steud.), Kisyuusuzumenohie (*Paspalum distichum* L.), Shima-suzumenohie (Dallis grass, *Paspalum dilatatum* Poir.)
Genus *Digitaria*: Mehishiba (*Digitaria ciliaris* (Retz.) Koeler), Akimehishiba (*Digitaria violascens* Link)
Genus *Pennisetum*: Chikarashiba (*Pennisetum alopecuroides* (L.) Spreng.), napier grass (*Pennisetum purpureum* Schumach.)
Genus *Spinifex*: Tsukiige (*Spinifex littoreus* (Burm.f.) Merr.)
Genus *Saccharum*: sugarcane (*Saccharum officinarum* L.), Waseobana (*Saccharum spontaneum* L. var. *arenicola* (Ohwi) Ohwi)
Genus *Imperata*: Chigaya (*Imperata cylindrica* (L.) Raeusch.)
Genus *Eccoilopus*: Aburasusuki (*Eccoilopus cotulifer* (Thunb.) A.Camus)
Genus *Miscanthus*: Susuki (Japanese pampas grass, *Miscanthus sinensis* Andersson), Ogi (*Miscanthus sacchariflorus* (Maxim.) Benth.), Tokiwasusuki (*Miscanthus floridulus* (Labill.) Warb. ex K.Schum. et Lauterb.), Kariyasu (*Miscanthus tinctorius* (Steud.) Hack.)
Genus *Microstegium*: Sasagaya (*Microstegium japonicum* (Miq.) Koidz.), Ashiboso (*Microstegium vimineum* (Trin.) A.Camus)
Genus *Pogonatherum:* Itachigaya (*Pogonatherum crinitum* (Thunb.) Kunth)
Genus *Dimeria*: Karimatagaya (*Dimeria ornithopoda* Trin.)
Genus *Hemarthria*: Ushinoshippei (*Hemarthria sibirica* (Gandog.) Ohwi), Kobanoushinoshippei (*Hemarthria compressa* (L.f.) R.Br.)
Genus *Phacelurus*: Aiashi (*Phacelurus latifolius* (Steud.) Ohwi)
Genus *Ischaemum*: Kamonohashi (*Ischaemum aristatum* L. var. *crassipes* (Steud.) Yonek.), Kekamonohashi (*Ischaemum anthephoroides* (Steud.) Miq.)
Genus *Arthraxon*: Kobunagusa (*Arthraxon hispidus* (Thunb.) Makino)
Genus *Cymbopogon*: Ogarukaya (*Cymbopogon tortilis* (J.Presl) Hitchc. var. *goeringii* (Steud.) Hand.-Mazz.)
Genus *Bothriochloa*: Hime-aburasusuki (*Capillipedium parviflorum* (R.Br.) Stapf)
Genus *Schizoachyrium*: Ushikusa *(Schizachyrium brevifolium* (Sw.) Nees ex Buse)
Genus *Andropogon*: Meriken-karukaya *(Andropogon virginicus* L.)
Genus *Themeda*: Megarukaya (*Themeda triandra* Forssk. *var. japonica* (Willd.) Makino)
Genus *Sorgham*: sorghum (*Sorghum bicolor* (L.) Moench), Sato-morokoshi (*Sorghum bicolor* (L.) Moench 'Dulciusculum'), Seiban-morokoshi (*Sorghum halepense* (L.) Pers.), Sudan grass (*Sorghum* x *drummondii* (Nees ex Steud.) Millsp. et Chase)
Genus *Coix*: Juzudama (*Coix lacryma-jobi* L.), Hatomugi (job's tears, *Coix lacryma-jobi* L. var. *ma-yuen* (Roman.) Stapf)
Genus *Zea*: maize

Tricin may be separated and purified by subjecting leaf, stem or the like of the above-described plants to extraction with suitable solvent, and by using separation and purification means such as HPLC. For example, tricin may be purified by concentrating a water extract, extracting the solid residue with alcohol or aqueous alcohol (e.g. methanol, ethanol, aqueous methanol or aqueous ethanol), dissolving the solid content in water, and by partitioning the resulting solution with ethyl acetate.
In 50 mL of various organic solvents, 5 g of Kumazasa dried leaf was immersed and the resulting mixture was left to stand, followed by concentrating the liquid extract to obtain solid content. The ratio of the solid content in various liquid extract and the ratio of tricin in the obtained solid content taking the weight of Kumazasa leaf as 100 are shown in Table below.

| Solvent | Ratio of Solid Content in Extract (%) | Ratio of Tricin in Solid Content (%) |
|---|---|---|
| Ethanol | 4.2 | 0.048 |
| Methanol | 11.7 | 0.035 |
| n-Butanol | 2.4 | 0.057 |
| Isopropanol | 2.2 | 0.070 |
| Ethyl Acetate | 1.9 | 0.065 |
| Acetone | 1.8 | 0.095 |
| Hexane | 1.3 | 0 |

Next, 50 g of Kumazasa leaf was extracted with 1 L of solvent, and the solvent in extract was evaporated to obtain a solid content. The amount of tricin (mg) contained in the solid content was measured. The results are shown in Table below.

| Solvent | Ratio of Tricin in Solid Content (mg) |
|---|---|
| Ethanol | 2.02 |
| Methanol | 4.10 |
| n-Butanol | 1.37 |
| Isopropanol | 1.54 |
| Ethyl Acetate | 1.62 |
| Acetone | 1.71 |
| Hexane | 0 |

In 500 mL of methanol, 50 g of Kumazasa leaf was immersed, and the resulting mixture was left to stand. Thereafter, the mixture was filtered and the solvent in the filtrate was evaporated to obtain 5.05 g of methanol extract. Partition operation was carried out by using hexane, chloroform, ethyl acetate and n-butanol in the order mentioned. The amount of the solid content and the amount of tricin therein were measured. The results are shown in Table below.

| Solvent | Ratio of Solid Content in Extract (%) | Ratio of Tricin in Solid Content (%) |
|---|---|---|
| Hexane | 17.6 | 0 |
| Chloroform | 4.7 | 0.61 |
| Ethyl Acetate | 6.9 | 0.09 |
| n-Butanol | 38.4 | 0 |
| Aqueous Layer | 33.3 | 0 |

Next, 50 g of Kumazasa leaf was extracted with 500 mL of methanol. The solvent in extract was evaporated and the resultant was dissolved in water, followed by partition operation by using various solvents. The amount of tricin in the obtained solid content was measured. The results are shown in Table below.

| Solvent | Ratio of Tricin in Solid Content (%) |
|---|---|
| Hexane | 0 |
| Chloroform | 3.35 |
| Ethyl Acetate | 0.73 |
| n-Butanol | 0 |
| Aqueous Layer | 0 |

Various aqueous ethyl alcoholic extracts of Kumazasa leaf were concentrated to dryness to obtain a solid content (B). After dissolving the solid content (B) in water, partition operation was carried out by using ethyl acetate, and the solvent in the resultant was evaporated to obtain a solid content (C). The ratio of the solid content (C) in ethyl acetate extract taking the solid content (B) as 100 is shown in Table below. The results of the analysis for tricin (% by weight) in the solid content (B) and in the solid content (C) are also shown therein.

| Ratio of Alcohol in Aqueous Alcohol (% by weight) | Solid Content (B) (% by weight) | Tricin in (B) (% by weight) | Solid Content (C) (% by weight) | Tricin in (C) (% by weight) |
|---|---|---|---|---|
| 0* | 62.0 | 0.025 | 5.5 | 0.38 |
| 5 | 3.0 | 0.010 | 7.7 | 0.057 |
| 10* | 51.6 | 0.031 | 7.1 | 0.29 |
| 15 | 3.1 | 0.043 | 11.1 | 0.12 |
| 25 | 3.2 | 0.15 | 12.2 | 0.77 |
| 30* | 55.4 | 0.092 | 11.4 | 0.80 |
| 70 | 4.9 | 0.30 | 26.5 | 1.1 |
| 90 | 3.9 | 0.45 | 33.3 | 0.95 |
| 100* | 23.8 | 0.11 | 37.4 | 0.51 |

| | | | | |
|---|---|---|---|---|
| *: Preliminary concentration was carried out. | | | | |

Various aqueous ethyl alcohol extracts (D) of leaf or stem of reed and leaf of bamboo (Take) were prepared, and the solvent in the extract was evaporated to obtain a solid content (E). The solid content (E) was dissolved in water, and partition operation was carried out by using ethyl acetate to obtain a solid content (F) in an ethyl acetate-soluble portion. The amount of tricin in the solid content (E) and in the solid content (F) when 100 g of the solid content (E) was used was analyzed. The results are shown in Table below.

| Extract (D) | Amount of Tricin in 100 g of Solid Content (E) (mg) | Amount of Tricin in 100 g of Solid Content (F) (mg) |
|---|---|---|
| Reed Leaf (25% ethanol) | 31 | 45.8 |
| Reed Stem (25% ethanol) | 28 | 19.6 |
| Take Leaf (70% ethanol) | 110 | 64.0 |
| Take Leaf (90% ethanol) | 150 | 109.2 |

### Microbicidal Composition of the Present Invention

The microbicidal agent according to the present invention which comprises as an effective ingredient at least one selected from the above-described compounds (vanillin, p-hydroxybenzaldehyde, *trans*-p-coumaric acid, 3-hydroxypyridine, 5,7,4'-trihydroxy-3',5'-dimethoxyflavone) (herein also referred to as "compound of the invention") may be used in various forms. For example, the agent of the present invention is suitable for a mucosal-protective composition, prophylactic and/or therapeutic composition for bacterial infection, preservative, and a microbicidal agent for a filtration apparatus or the like.
The microbicidal agent according to the present invention may be formulated in the form of liquid, solid or gas. The microbicidal agent according to the present invention may be administered either orally or parenterally. Examples of the oral administration forms include tablets, balls, powders, liquids, and food forms such as chewing gums, candies, chocolates, bread, cookies, buckwheat noodles, Japanese wheat noodles, various drinkable preparations and the like. Examples of the parenteral administration forms include injection solutions, formulations for topical administration (such as creams, ointments), suppositories and the like. Examples of the formulation for topical administration include carriers such as gauzes made of natural or synthetic fibers in which carriers the microbicidal agent of the present invention is impregnated, and cosmetics such as lipsticks and the like in which the microbicidal agent of the present invention is incorporated.
The microbicidal agent of the present invention may be useful as a microbicidal agent for not only human but also other animals such as mammals, birds, fishes, reptiles and so on. Therefore, the agent of the present invention may be used as a microbicidal agent for these animals (for example, a pharmaceutical for pets, pet food or the like). Moreover, the microbicidal agent of the present invention is useful as the agent for various plants as well as animals, and also useful as a preservative.

For the production of the microbicidal agent of the present invention in various formulations, base materials such as oily components used in usual pharmaceutical compositions, cosmetics, compositions for skin and the like; moisturizers; preservatives and the like, in addition to a prescribed amount of the above-described compound, may be used.
Water used in the microbicidal agent is not restricted, and it may be tap water, natural water, purified water or the like. In general, water with a high purity such as ion exchanged water is preferably used.
Examples of the oily component include animal oils such as squalane, beef tallow, lard, horse oil, lanolin, beeswax and the like; plant oils such as olive oil, grape seed oil, palm oil, jojoba oil, germ oil (e.g. rice germ oil) and the like; synthetic oils such as liquid paraffin, higher fatty acid ester (e.g. octyl palmitate, isopropyl palmitate, octyldodecyl myristate), silicone oil and the like; and semisynthetic oils.
Two or more of the oily components are used appropriately in combination depending on what is desired, for example, protection of skin, emollient effect (to cover the surface of the skin with thin layer to protect skin from drying, and to give the skin tenderness and elasticity), light texture and so on. One of the preferred examples is a combination of squalane, olive oil and octyldodecyl myristate.
To adjust hardness and/or fluidity of the microbicidal agent, solid fats such as stearic acid, stearyl alcohol, behenic acid, cetanol, vaseline and the like may be used. Preferably, stearic acid and cetanol are used in combination.

To produce the microbicidal agent of the present invention as a cream composition, a creaming agent which makes a mixture of the compound of the invention, water and oily component creaminess is used. The creaming agent is not restricted, and in general a combination of glyceryl monostearate and self-emulsifying glyceryl monostearate (a mixture of glyceryl monostearate and emulsifier) is used.
The microbicidal agent of the present invention may comprise (a) stabilizer(s), moisturizer(s), wound healing agent(s), preservative(s), surfactant(s) or the like as required.
Examples of the stabilizer include a combination of carboxyvinyl polymer and potassium hydroxide, polyethylene glycol distearate and the like. Especially, polyethylene glycol sesquistearate (a 1:1 mixture of polyethylene glycol distearate and polyethylene glycol monostearate) (a molecular weight of polyethylene glycol is 1,000 to 20,000) is preferred, because it has a high stability and the composition comprising it does not separate into water and oil, and moreover it can adjust effectively the hardness of the cream composition which is applied to skin.
Examples of the moisturizer include sodium hyaluronate, collagen, aloe extract (especially preferred is an aloe extract (2) originated from *Aloe arborescens* var. *natalensi*), urea, 1,3-butylene glycol, glycerin, trehalose, sorbitol, amino acid, sodium pyrrolidone carboxylate and the like.
Examples of the wound healing agent include allantoin, dipotassium glycyrrhizinate, licorice extract, Artemisia extract and the like.

Preservatives are optionally used because the compound of the invention itself has a microbicidal effect. Examples of the preservative include sodium benzoate, lower alkyl esters of para-hydroxybenzoic acid (e.g. esters called paraben such as methyl, ethyl, propyl or butyl ester), sodium propionate, mixed fatty acid ester (a mixture of glyceryl caprate, polyglyceryl-2 laurate and polyglyceryl-10 laurate), phenoxyethanol, photosensitive pigment No. 201 (yellow pigment), 1,2-pentanediol and the like, and preferred are paraben, mixed fatty acid ester and 1,2-pentanediol.
Examples of the surfactant include sodium N-acyl-L-glutamate, polyoxyethylene sorbitan monostearate and the like.
Further as required, perfume components such as orange oil, lemon oil, bitter orange oil, flavoring agents and the like may be contained.

A mucosal-protective composition, prophylactic and/or therapeutic composition for bacterial infection which comprise the microbicidal agent of the present invention are the agents which effectively suppress invasion of infectious bacteria into a body through a mucosa of eye, nose, throat, ear, anus, genital organs or the like, and through wounds and skin, thereby preventing and/or treating bacterial infection including nosocomial infection. More concrete examples of the mucosal-protective composition and prophylactic and/or therapeutic composition for bacterial infection include mucosal-protective cloths and compositions for oral cavity application.

A mucosal-protective cloth is an air-permeable carrier in which the microbicidal agent of the present invention is incorporated by a method such as impregnation, spraying or the like, wherein the air-permeable carrier is, for example, natural fibers such as silk, cotton, hemp and the like, synthetic fibers such as polyurethane, polyethylene, polypropylene, nylon, polyester, acryl and the like, semi-synthetic fibers, or mixed fibers of two or more of these fibers; or yarns, woven fabric, knit, nonwoven fabric or paper thereof. The term "protective cloth" herein includes not only cloths but also fibers *per se,* yarns, papers and the like for convenience.
Concrete examples of the form of the mucosal-protective cloth include not only protective cloths directly contacting with mucosa or skin which are classified as sanitary goods such as gauzes, masks, eye patches, sanitary belts, sanitary napkins, bandages, toilet papers, gauzes for hemorrhoids treatment, earplugs, liquid bandages and the like, but also articles contacting directly or indirectly with skin, e.g., clothes such as white robes and the like; small clothing articles such as gloves, hats, socks, Japanese socks and the like; beddings such as bed sheets, quilt covers, pillowcases, articles for bedding and the like; interior accessories and interior finishing materials such as curtains, wallpapers, carpets and the like; medical materials such as surgical sutures and the like, and so on.

Examples of the composition for oral cavity application comprising the microbicidal agent of the present invention include gummies, jelly, troches, candies, chewing gums, tablets, balls, mouth washes, collutories, tooth pastes, films for application to mucosa, nebulae for treatment of pharyngeal inflammation and the like.

As shown in the above-described experimental examples, the compound of the invention exists in Kumazasa extract at a concentration of about 1/1,000 to 1/100,000 with respect to the solid content therein. Therefore, in cases where the compound of the invention is used as a microbicidal agent, the compound may be used at a concentration of about 1/1,000 to 1/100,000 of an used amount of the solid content of Kumazasa extract. The concentration may be appropriately determined depending on the purpose of use.
For example, a mucosal-protective cloth in which the compound of the invention is incorporated may be obtained by, for example, impregnating a protective cloth in 2 to 20 x (1/1,000 to 1/100,000) wt% solution of the compound of the invention (in water, an organic solvent such as alcohol or DMSO, or mixture thereof), or by spraying a protective cloth with 2 to 20 x (1/1,000 to 1/100,000) wt% solution of the compound of the invention and then drying it. The amount of the compound of the invention used in impregnation or spraying is, preferably about 2 to 20 x (1/1,000 to 1/100,000) wt%, more preferably about 6 to 15 x (1/1,000 to 1/100,000) wt%, most preferably about 8 to 12 x (1/1,000 to 1/100,000) wt% in terms of solid content of the compound of the present invention.

When the compound of the present invention is incorporated in compositions for oral cavity application such as gummies, jelly, troches, candies, chewing gums, tablets, balls (for example sasatan), mouth washes, collutories, tooth pastes, films for application to mucosa and the like, the compound of the invention may be added to the source material of the composition for oral cavity application in an amount of about 2 to 20 x (1/1,000 to 1/100,000) wt%, more preferably about 6 to 15 x (1/1,000 to 1/100,000) wt%, most preferably about 8 to 12 x (1/1,000 to 1/100,000) wt% in terms of solid content of the compound of the invention in any one of the steps for producing the composition for oral cavity application.

Commonly used component(s) may be appropriately blended to the composition for oral cavity application of the present invention, depending on its formulation.
Examples of the commonly used component include excipients such as glucose, lactose, sucrose, starch syrup, dextrin, cyclodextrin and the like; binders such as gum arabic, sodium carboxymethylcellulose, crystalline cellulose, gum base and the like; disintegrators such as starch and the like; lubricants such as magnesium stearate, sucrose fatty acid esters and the like; refrigerants such as perfumes, chlorophyll, peppermint, 1-menthol and the like, and so on.

In cases where the mucosal-protective composition according to the present invention is used in the form of a protective cloth, the compound of the invention may be incorporated in a part of a protective cloth (e.g. gauze) with which part mucosa or wound contacts, and the protective cloth may be changed to a fresh one once to three times a day. When the mucosal-protective composition is used in the form of a surgical sutures, invasion of bacteria through the sutured area can be effectively suppressed and the inflammation of the affected area can be suppressed, thereby promoting recovery of the operative site.
When the mucosal-protective composition is used in the form of a protective cloth in order to prevent nosocomial infection in a hospital or the like, the effect of the mucosal-protective composition of the invention to prevent infection continues for a long time. If the effect is reduced by washing, the protective cloth may be appropriately changed, or the process for incorporating the compound of the invention in the protective cloth again may be performed.

In cases where the mucosal-protective composition of the present invention is used in the form of a composition for oral cavity application, the intake of the composition is not restricted, and the compound of the invention in an amount of about 2 to 15 x (1/1,000 to 1/100,000) mg in terms of solid content may be taken, for example, once to five times a day, usually once to three times a day. The intake and number of intake may be appropriately changed depending on the purpose.

The mucosal-protective composition of the present invention comprises the compound of the invention in an amount of 2 to 20 x (1/1,000 to 1/100,000) wt% in terms of solid content, and has a significant bactericidal effect even against the resistant bacteria to which conventional antibiotics are ineffective.
In cases where the mucosal-protective composition of the present invention is used in the form of a composition for oral cavity application, infections by infectious bacteria can be prevented and the growth thereof can be suppressed very simply by appropriately placing the composition into mouth as required. The composition in sustained release form such as chewing gum, candy or the like can exert its effect for a long time and therefore such form is advantageous.
The mucosal-protective composition of the present invention may be used in the form of lotions or oils. By applying to skin a lotion or oil comprising the compound of the invention in an amount of 2 to 20 x (1/1,000 to 1/100,000) wt% in terms of solid content, infections of infectious bacteria can be prevented and their growth can be suppressed very simply.

### Preservatives

The present invention further provides a preservative comprising as an effective ingredient the compound of the invention. The form of the preservative is not restricted. Examples of the composition to which the preservative of the present invention is applied include foodstuffs, drinking water, condiments, cosmetics (including lotions and oils), nebulae for treatment of wound, nebulae for treatment of pharyngeal inflammation and the like. These composition may appropriately comprise the compound of the invention in an amount of 2 to 20 x (1/1,000 to 1/100,000) wt%, more preferably about 6 to 15 x (1/1,000 to 1/100,000) wt%, most preferably about 8 to 12 x (1/1,000 to 1/100,000) wt% in terms of solid content.

### Filtration Apparatus

The present invention also provides an air filtration apparatus comprising as an effective ingredient the compound of the invention. Examples of the concrete form of the apparatus include filters which are applied to a region through which the air passes, for example, filters used in ventilation fans, air-conditioners, car air-conditioners, air admission ports, air exhaust ports, screen doors, air cleaners and the like. The material constituting the filter is not restricted and examples of the material include woven fabrics, knits, nonwoven fabrics, papers and the like, which are made of natural fibers such as silk, cotton, wool, hemp and the like, synthetic fibers such as polyurethane, polyethylene, polypropylene, nylon, polyester, acryl and the like, semi-synthetic fibers, or mixed fibers of two or more of these fibers. The compound of the invention may be incorporated in such a filter by a method such as impregnation, spraying or the like, and the filter may be dried. The amount of the compound of the present invention contained in the filter is preferably 2 to 20 x (1/1,000 to 1/100,000) wt%, more preferably about 6 to 15 x (1/1,000 to 1/100,000) wt%, most preferably about 8 to 12 x (1/1,000 to 1/100,000) wt% in terms of solid content.

## Claims

1. A microbicidal agent comprising as an effective ingredient at least one selected from the group consisting of p-hydroxybenzaldehyde, 5,7,4'-trihydroxy-3',5'-dimethoxyflavone, 3-hydroxypyridine, and vanillin.

2. The microbicidal agent according to claim 1, wherein said microorganism is selected from the group consisting of *Streptococcus, Enterococcus, Staphylococcus, Escherichia, Salmonella, Yersinia, Vibrio, Pseudomonas, Bacillus,* and *Candida.*

3. A microbicidal agent comprising as an effective ingredient at least one selected from the group consisting of p-hydroxybenzaldehyde, and 5,7,4'-trihydroxy-3',5'-dimethoxyflavone.

4. The microbicidal agent according to claim 3, wherein said microorganism is selected from the group consisting of *Streptococcus, Enterococcus, Staphylococcus, Escherichia, Salmonella, Yersinia, Vibrio, Pseudomonas, Bacillus,* and *Candida.*

5. The microbicidal agent according to claim 1, wherein said agent comprises 3-hydroxypyridine as an effective ingredient, and said microorganism is selected from the group consisting of *Streptococcus pneumoniae, Streptococcus pyogenes, Staphylococcus aureus, Yersinia enterocolitica, Pseudomonas aeruginosa,* and *Candida* sp..

6. The microbicidal agent according to claim 1, wherein said agent comprises p-hydroxybenzaldehyde as an effective ingredient, and said microorganism is selected from the group consisting of *Streptococcus pneumoniae, Streptococcus pyogenes, Staphylococcus aureus, Escherichia coli, Salmonella* sp., *Yersinia enterocolitica, Vibrio parahaemolyticus, Pseudomonas aeruginosa, Bacillus cereus, Bacillus subtilis,* and *Candida* sp..

7. The microbicidal agent according to claim 1, wherein said agent comprises vanillin as an effective ingredient, and said microorganism is selected from the group consisting of *Streptococcus pneumoniae, Streptococcus pyogenes, Staphylococcus aureus, Escherichia coli, Salmonella* sp., *Yersinia enterocolitica, Vibrio parahaemolyticus, Bacillus cereus, Bacillus subtilis,* and *Candida* sp..

8. The microbicidal agent according to claim 1, wherein said agent comprises 5,7,4'-trihydroxy-3',5'-dimethoxyflavone as an effective ingredient, and said microorganism is selected from the group consisting of *Salmonella* sp., and *Pseudomonas aeruginosa.*

9. A microbicidal composition comprising the microbicidal agent according to any one of claims 1 to 8.
